# EUROPEAN PATENT APPLICATION

(11) **EP 3 627 517 A1**
(43) Date of publication of application: **25.03.2020**
(21) Application number: 18196214.3
(22) Date of filing: 24.09.2018
(51) Int. Cl.: G16H 40/63

(54) **MEDICAL MONITORING SYSTEM**

(71) Applicant: Koninklijke Philips N.V., 5656 AG Eindhoven (NL)
(72) Inventor: GREINER, Harald, 5656 AE Eindhoven (NL)
(74) Representative: de Haan, Poul Erik

(57) **Abstract**

The invention relates to a medical monitoring system controller (10) for controlling a medical monitoring system (1). The controller is configured to control the medical monitoring system such that the temporal resolution of storing measured medical parameters like blood pressure, heart rate et cetera in a storing unit (8) depends on the identification of a user like a physician or nurse, wherein the identification is preferentially an authentication of the user. In particular, the temporal resolution of storing measured medical parameters can be increased, if a user has been identified. It is therefore not required to store the medical parameter with a relatively high temporal resolution all the time, but this temporal resolution might generally be relatively low and increased, if a user has been identified. This allows for a significant reduction of the required storage capacity.

## Description

### FIELD OF THE INVENTION

The invention relates to a medical monitoring system for monitoring a subject. The invention further relates to a medical monitoring system controller, a control method and a computer program for controlling the medical monitoring system.

### BACKGROUND OF THE INVENTION

In the field of medical monitoring many medical parameters are generally measured like the heart rate, the breathing rate, the blood pressure, the oxygen saturation or other vital signs. Especially in hospitals with often thousands of patients a huge amount of data is thereby generated, which need to be stored, in order to be able to, for instance, decide what exactly happened during certain times where significant events occurred. This can be particularly important in situations in which a patient dies. Storing the huge amount of medical parameter data requires a relatively large data storage capacity.

### SUMMARY OF THE INVENTION

It is an object of the present invention to provide a medical monitoring system for monitoring a subject, a medical monitoring system controller, a control method and a computer program for controlling the medical monitoring system, which allow for a reduced required storage capacity.

In a first aspect of the present invention a medical monitoring system controller for controlling a medical monitoring system for monitoring a subject is presented, wherein the medical monitoring system comprises a medical parameter measuring unit configured to measure a medical parameter of the subject over time, an identification unit configured to identify a user and a storing unit configured to store the measured medical parameter, wherein the medical monitoring system controller is configured to control the medical monitoring system such that the temporal resolution of storing the measured medical parameter in the storing unit depends on the identification of a user.

By modifying the temporary resolution of storing the measured medical parameter in the storing unit depending on the identification of a user, the amount of data stored at a certain time can be adapted to whether a certain user is present or not. For instance, the medical monitoring system controller can be configured to control the medical monitoring system such that the temporal resolution of storing the medical parameter is increased, if a user has been identified. It is also possible that the medical monitoring system controller is configured to compare, if a user has been identified, the identified user with a list of users, thereby generating a comparison result, and to control the medical monitoring system such that the temporal resolution of storing the measured medical parameter in the storing unit depends on the comparison result, wherein the temporal resolution of storing the measured medical parameter can be increased, if the comparison result indicates that the identified user is on the list of users. It is therefore not required to store the medical parameter measured over time with a relatively high temporal resolution all the time, but this temporal resolution might generally be relatively low and increased, if a user has been identified, especially if the identified user is on a predefined list of users. This allows for a significant reduction of the required storage capacity.

The identification unit is preferentially configured to authenticate a user for identifying the user. Thus, preferentially the identification is an authentication of a user, and in a preferred embodiment the identification unit could also be regarded as being an authentication unit. In particular, the controller is configured to control the medical monitoring system such that the temporal resolution of storing the measured medical parameter in the storing unit depends on the authentication of a user. The term *"identification* " can hence refer to a simple identification without authentication, but it preferentially refers to an authentication which inherently includes a simple identification. For instance, a simple identification can be regarded as being the ability to identify uniquely a user, for example, by using a user ID, i.e. the identification unit may just request the user to input a user ID for allowing the identification unit to uniquely identify the user. However, if the identification unit is configured to authenticate the user, in this example the identification unit also requests a password and checks whether the password is correct for the input user ID, in order to authenticate the user. The authentication can hence allow the identification unit to prove that a user is genuinely who that person claims to be and/or to prove that a user is really the person who the identification unit initially believes he/she is. Although in the above example the identification unit is configured to use a user ID and a password for authenticating and thereby also identifying the person, the identification unit can of course also be configured to use other known authentication means.

The medical monitoring system controller can comprise inputs and outputs for communicating with the different components of the medical monitoring system. For instance, it can comprise an input for receiving a signal from the identification unit, which indicates that a user has been identified, particularly authenticated. It might be possible that the medical monitoring system controller is in a same casing together with the medical parameter measuring unit and/or the storing unit, wherein the identification unit can be in another casing. For example, the identification unit can be a separate device, which can be located next to a casing comprising the medical parameter measuring unit, the storing unit and the medical monitoring system controller, wherein the medical monitoring system controller can comprise an input to which the identification unit can be connected, possibly via a connector at the outside of the casing, in order to allow the identification unit to provide a corresponding signal to the medical monitoring system controller. However, the different components of the medical monitoring system can also be distributed in another way. For example, they can all be included in a single casing and form a single device, they can be distributed among several rooms in a hospital, et cetera.

In an embodiment the identification unit, the storing unit and the medical monitoring system controller are included in a first device, i.e., for instance, in a first casing, wherein the medical parameter measuring unit is or part of a second device being spatially separated from the first device. In particular, the first device and the second device can be located adjacent to each other, wherein the first device and the second device can be adapted to allow for a data connection between these two devices, in order to allow for a control of the medical monitoring system comprising the two devices such that the temporal resolution of storing the measured medical parameter in the storing unit depends on the identification of a user.

The medical parameter measuring unit can be adapted to measure one or several medical parameters. If a current temporal resolution of measuring one or several medical parameters to be stored is too low such that the one or more medical parameters cannot be stored with a required increased temporal resolution, the medical monitoring system controller controls the medical parameter measuring unit such that the temporal resolution of measuring the one or several medical parameters is increased accordingly.

In an embodiment the medical monitoring system controller is configured to determine whether the measured medical parameter fulfils a predefined condition and to control the medical monitoring system such that the temporal resolution of storing the measured medical parameter in the storing unit depends on the fulfilment of the predefined condition. In particular, the medical parameter measuring unit is configured to measure several medical parameters over time, thereby generating several medical parameter waveforms, wherein the medical monitoring system controller is configured to combine the several medical parameter waveforms for generating a combination result, to determine whether the combination result fulfils a predefined condition and to control the medical monitoring system such that the temporal resolution of storing the measured medical parameters in the storing unit depends on the fulfilment of the predefined condition. For instance, if the predefined condition is fulfilled, the temporal resolution of storing the measured medical parameter can be increased. In this embodiment the temporal resolution of storing the measured medical parameters does not only depend on the identification of a user, but also on the measured medical parameters. This can further ensure that, although generally the measured medical parameter is stored with a relatively low temporal resolution, at medically relevant events, the temporal resolution of storing the measured medical parameter is nevertheless relatively high.

In an embodiment the medical monitoring system controller is configured to control the medical monitoring system such that the temporal resolution of measuring the medical parameter depends on the identification of a user. In particular, the medical monitoring system controller can be configured to control the medical monitoring system such that the temporal resolution of measuring the measured medical parameter is increased, if a user has been identified. In an embodiment the medical parameter measuring unit comprises a camera for acquiring images of the subject over time, wherein the medical parameter measuring unit is configured to determine the medical parameter based on the acquired images, wherein the medical monitoring system controller is configured to control the medical monitoring system such that the frequency of acquiring the images and hence the temporal resolution of measuring the medical parameter depends on the identification of a user. The camera can be a camera, which is adapted to acquire single images and/or it can be a camera, which acquires a video, i.e. several images with a relatively low temporal distance. The medical parameter measuring unit can be configured to determine, for instance, the heart rate and/or the breathing rate by sensing changes in skin color and/or body movements. The camera can be configured to be operated in the infrared wavelength range and/or in the visible wavelength range. If the medical parameter is determined based on the acquired image, the frequency of acquiring the images can depend on the identification of the user and optionally also on whether the medical parameter fulfils a predefined condition. For instance, the frequency of acquiring the images, i.e. in the case of a video, the frame rate, can be increased, if a user has been identified and optionally if the measured medical parameter fulfils a predefined condition, in order to allow for an increased temporal resolution of storing the medical parameter which is determined based on the acquired images.

It is preferred that the medical monitoring system further comprises a medical report generation unit configured to generate a medical report including the measured medical parameter, wherein the storing unit is configured to store the medical parameter by storing the medical report, wherein the medical monitoring system controller is configured to control the medical monitoring system such that the temporal resolution of the medical parameter included in the medical report depends on the identification of a user.

In a further aspect of the present invention a medical monitoring system for monitoring a subject is presented, wherein the medical monitoring system comprises:
- a medical parameter measuring unit configured to measure a medical parameter of the subject over time,
- an identification unit configured to identify a user,
- a storing unit configured to store the measured medical parameter,
- a medical monitoring system controller configured to control the medical monitoring system as defined by claim 1.

The medical parameter measuring unit is preferentially configured to measure vital signs as medical parameters. In particular, the medical parameter measuring unit is configured to measure at least one medical parameter of a list consisting of the heart rate, the blood pressure, the oxygen saturation and the breathing rate.

In another aspect of the present invention a control method for controlling a medical monitoring system for monitoring a subject as defined by claim 11 is presented, wherein the medical monitoring system is controlled such that it carries out:
- measuring a medical parameter of the subject over time by a medical parameter measuring unit,
- identifying a user by an identification unit, and
- storing the measured medical parameter by a storing unit depending on the identification of a user.

In an aspect of the present invention a computer program for controlling a medical monitoring system for monitoring a subject as defined by claim 11 is presented, wherein the computer program comprises program code means for causing the medical monitoring system to carry out the steps of the control method as defined in claim 13, when the computer program is run on the medical monitoring system controller controlling the medical monitoring system.

It shall be understood that the medical monitoring system controller of claim 1, the medical monitoring system of claim 11, the control method of claim 13, and the computer program of claim 14, have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of a medical monitoring system for monitoring a subject,
Fig. 2 shows schematically and exemplarily a further embodiment of a medical monitoring system for monitoring a subject,
Fig. 3 shows schematically and exemplarily an even further embodiment of a medical monitoring system for monitoring a subject.
Fig. 4 shows a flowchart exemplarily illustrating an embodiment of a control method for controlling a medical monitoring system for monitoring a subject.

### DETAILED DESCRIPTION OF EMBODIMENTS

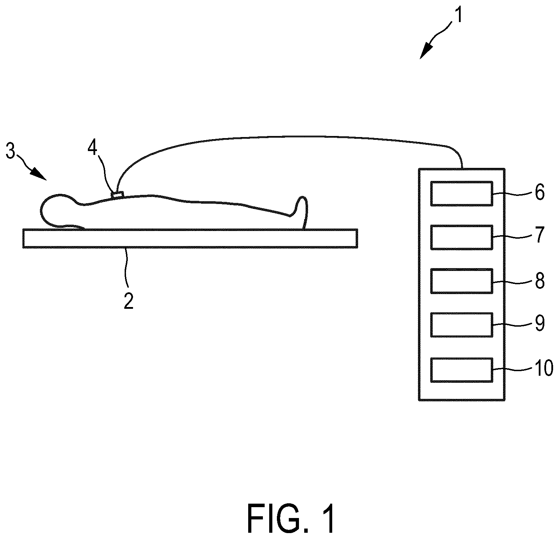

Fig. 1 shows schematically and exemplarily an embodiment of a medical monitoring system for monitoring a subject. In this embodiment the subject is a patient 3 lying in a bed 2, wherein the medical monitoring system 1 comprises a medical parameter measuring unit 4, 6 configured to measure a medical parameter of the subject over time.

The medical parameter measuring unit can include a detector or sensor 4 for generating a detection signal depending on a vital sign being, in this embodiment, the medical parameter, wherein the detector 4 might be an electrical detector, an optical detector, a magnetic detector, an ultrasound sensor or another type of detector. The detector 4 can comprise, for instance, an electrode like an electrocardiography (ECG) electrode, an optical photoplethysmography (PPG) detector and/or an ultrasound sensor array. The medical parameter measuring unit further comprises a processing unit 6 for processing the detection signal received from the detector 4 and for determining a value being indicative of the measured medical parameter. The medical parameter measuring unit can be adapted to derive one or several medical parameters from the acquired detection signals like the heart rate, the breathing rate, the blood pressure, the oxygen saturation, et cetera.

It should be noted that in Fig. 1 the position of the detector 4 on the patient 3 might not be the real position in a real situation, i.e. the position of the detector 4 of course depends on the kind of detector used for measuring the medical parameter. For instance, if the detector comprises an ECG electrode, it might be located on the chest of the patient 3, and, if the detector 4 is a PPG detector, it might be positioned at a finger of the patient 3.

The medical monitoring system 1 further comprises an identification unit 7 configured to authenticate a user. The user to be authenticated is, for instance, a physician or a caregiver like a nurse. The identification unit 7 can be configured to use known authentication means like the use of passwords, RFID chips, ID cards, fingerprints, face recognition, et cetera.

The medical monitoring system 1 also comprises a storing unit 8 configured to store the measured medical parameter, a display 9 configured to display the measured medical parameter and a medical monitoring system controller configured to control the medical monitoring system 1. The medical monitoring system controller 10 is configured to control the medical monitoring system 1 such that the temporal resolution of storing the measured medical parameter in the storing unit 8 depends on the authentication of a user. In an embodiment the medical monitoring system controller 10 is configured to control the medical monitoring system 1 such that the temporal resolution of storing the measured medical parameter is increased, if a user has been authenticated. Moreover, in an embodiment the medical monitoring system controller is configured to compare, if a user has been authenticated, the authenticated user with a list of users, thereby generating a comparison result, and to control the medical monitoring system 1 such that the temporal resolution of storing the measured medical parameter is increased if the comparison result indicates that the authenticated user is on the list of users.

For example, an acquisition sample rate of a sensor providing signals for the heart rate derivation is about 250 Hz, while an acquisition sample rate of the PPG sensor is about 125 Hz. At these temporal resolutions a heart rate and SpO2 waveforms are visualized by the medical monitoring system in this example. A regular temporal resolution of these waveforms, at which they are stored in the storage unit for further reporting purposes, for example, is one averaged number per minute or 5 minutes. Once the user authentication event occurs the temporal resolution is increased to, for instance, one averaged number per minute or to a higher temporal resolution, to one averaged number per 30 seconds or to a higher resolution, or even to one averaged number per 15 seconds or to a higher resolution. Instead of or in addition to storing an averaged number it is also possible that non-averaged numbers are stored. In this example, preferentially, for each measured medical parameter an averaged number and/or a non-averaged number is stored with the increased temporal resolution, if a user has been authenticated.

The medical monitoring system controller 10 can also be configured to determine whether the measured medical parameter fulfils a predefined condition and to control the medical monitoring system 1 such that the temporal resolution of storing the measured medical parameter in the storing unit 8 depends on the fulfillment of the predefined condition. For instance, the medical parameter measuring unit 4, 6 can be configured to measure several medical parameters over time, thereby generating several medical parameter waveforms, wherein the medical monitoring system controller 10 can be configured to combine the several medical parameter waveforms for generating a combination result. The medical monitoring system controller 10 can then be further adapted to determine whether the combination result fulfils a predefined condition and to control the medical monitoring system 1 such that the temporal resolution of storing the measured medical parameters in the storing unit 8 depends on the fulfillment of the predefined condition. In particular, the temporal resolution of storing the measured medical parameters can be increased, if the predefined condition is fulfilled.

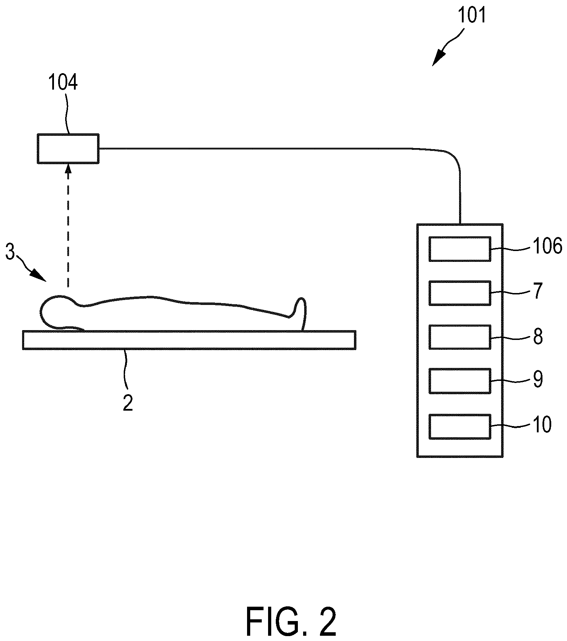

Fig. 2 shows schematically and exemplarily a further embodiment of a medical monitoring system for monitoring a subject. This embodiment of the medical monitoring system is similar to the embodiment described above with reference to Fig. 1, wherein, instead of the medical parameter measuring unit 4, 6, the medical monitoring system 101 shown in Fig. 2 comprises a camera 104 and a processing unit 106, which form the medical parameter measuring unit in this embodiment. The camera 104 is configured to acquire images of the subject over time, particularly a video of the subject 3, wherein the processing unit 106 is configured to determine the medical parameter based on the acquired images. Thus, in this embodiment the medical parameter like the heart rate or the breathing rate can be determined without needing to contact the subject 3. Moreover, in this embodiment the medical monitoring system controller 10 can be configured to control the medical monitoring system 101 such that the frequency of acquiring the images depends on the authentication of the user. For instance, if a user on the user list has been authenticated, the frequency of acquiring the images, i.e. the frame rate if the camera acquires a video, can be increased, in order to allow for an increased resolution of measuring the medical parameter and hence for an increased temporal resolution of storing the medical parameter. The processing unit 106 can be adapted to use the technique provided by the Vital Signs Camera of the company Philips for determining, for instance, the heart rate or the breathing rate based on the acquired images.

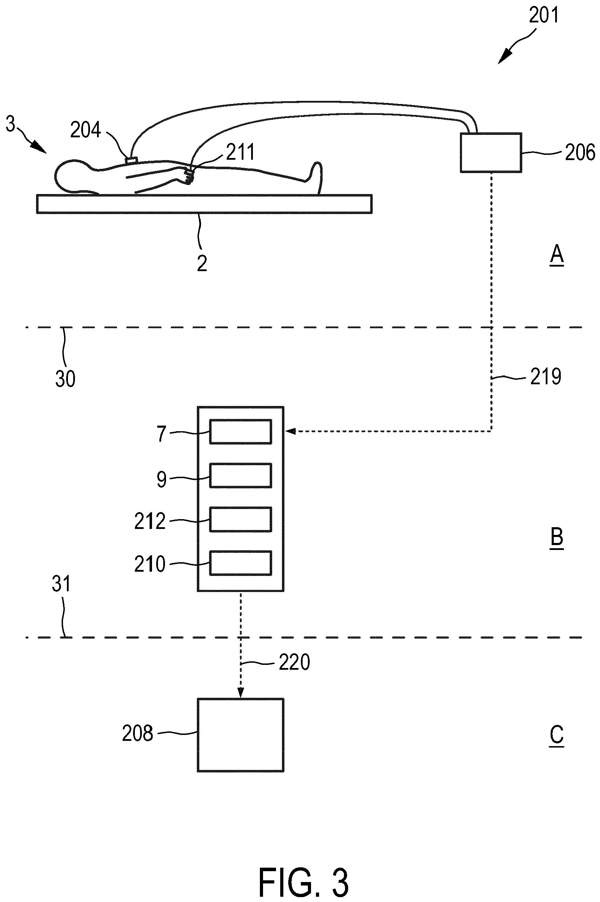

Fig. 3 schematically and exemplarily shows a further embodiment of a medical monitoring system for monitoring a subject. In this embodiment the medical monitoring system 201 comprises a medical parameter measuring unit 204, 206, 211 configured to measure medical parameters of the subject 3 over time in a patient room A. The medical parameter measuring unit comprises a first detector 204 for generating first detection signals being indicative of a first medical parameter of the patient 3 and a second detector 211 for generating a second detection signal being indicative of a second medical parameter of the patient 3. The detection signals are processed by a processing unit 206 for determining respective values of the respective medical parameter. For instance, the processing unit 206 can be configured to determine the heart rate, the respiratory rate, the blood pressures and/or the oxygen saturation based on the received detection signals. The first detector can comprise, for instance, an ECG electrode and the second detector 211 can be, for instance, a PPG detector. The measured medical parameters are transmitted via a data connection 219 to another room B which might be a room where the caregivers like nurses are normally located, if they are not in a patient room like the patient room A. In Fig. 3, the dashed lines 30, 31 illustrate borders like walls between the different rooms.

In the room B a further part of the medical monitoring system 201 is located. This further part comprises the identification unit 7 configured to authenticate a user, one or several displays 9 for showing the measured medical parameters, a medical report generation unit 212 configured to generate a medical report about the patient 3, which includes the measured medical parameters, and a medical monitoring system controller 210 for controlling the medical monitoring system 201. In this embodiment the storing unit 208 is configured to store the medical parameters by storing the medical report and it is located in a further room C, wherein the generated medical report is transmitted to the storing unit 208 via a data connection 220. In this embodiment the storing unit 208 can be regarded as being an electronic patient record database in which the medical reports can be stored.

The medical monitoring system controller 210 is configured to control the medical monitoring system 201 such that the temporal resolution of the medical parameters included in the medical report depends on the authentication of a user. Thus, if a user like a staff member of a hospital is authenticated by the identification unit 7 in the room B, this authentication event can trigger an e-report creation, i.e. a creation of the medical report by the medical report generation unit 212, with an increased temporal resolution, i.e. with a higher sampling rate, of medical parameters which preferentially include vital signs waveforms.

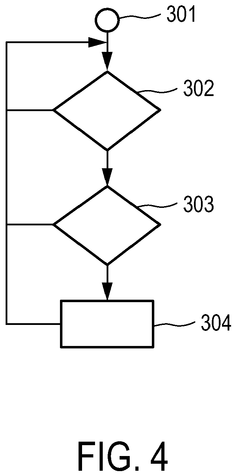

In the following an embodiment of a control method for controlling a medical monitoring system for monitoring a subject will exemplarily be described with reference to a flowchart shown in Fig. 4.

In step 301 the control method is started, wherein this includes starting a continuous measurement of medical parameters of a subject over time by one or several medical parameter measuring units. This measurement of the medical parameters is continuously carried out, while the following steps are performed. In step 302 it is determined whether an identification unit has authenticated a user. If this is the case, in step 303 it is further determined whether the authenticated user is a member of a predefined list of users, wherein, if also this is the case, in step 304 the temporal resolution of storing the measured medical parameters is increased. If the temporal resolution of the measurement of the medical parameters is not sufficient for storing the medical parameters with the increased temporal resolution, in step 304 also the temporal resolution of the measurement of the medical parameters is increased.

Thus, in this embodiment not any authentication of a user leads to an increased temporal resolution of storing the medical parameters, but the temporal resolution is only increased, if the authenticated user is in the predefined list of users. In another embodiment, step 303 can be omitted such that each authorization of a user leads to an increased temporal resolution of storing the medical parameters.

The patient monitoring systems and the control method described above with reference to Figs. 1 to 4 allow for a temporal high resolution storage, medical data input-wise, triggered by a predefined event during a patient's stay at a hospital, wherein the predefined event is at least an authentication of a user like a physician or a nurse. Storing all medical parameters obtained in a hospital with a rather deep and high resolution requires a relatively large storage capacity such that it would be advantageous, if the medical parameters measured over time for the often thousands of patients in a hospital could be stored with a lower resolution. However, in clinical settings it is sometimes important to be able to decide what exactly happened in certain situations with possibly extreme outcomes like a death of a patient. The medical monitoring systems and control method described above with reference to Figs. 1 to 4 allow to generally reduce the temporal resolution of storing the measured medical parameters, wherein, if a relevant situation occurs as indicated by the authentication of a user, the temporal resolution of storing the measured medical parameters is increased, in order to ensure that in relevant situations the temporal resolution is high enough.

The processing part of the medical parameter measuring unit like the processing unit 6, 106, 206, the identification unit, the storing unit, and the medical monitoring system controller can all be included in a single device which might be regarded as being a patient monitor as illustrated in Figs. 1 and 2. However, it is also possible that, for instance, the storing unit is not part of such a patient monitor, but an external device which is not included in the patient monitor which, in this example, includes the processing unit of the medical parameter measuring unit and optionally the identification unit and the medical monitoring system controller. Thus, the medical parameter to be stored can be made available to an external device which might nevertheless be regarded as being part of the overall patient monitoring system as exemplarily described above with reference to Fig. 3.

Fig. 3 illustrates an ecosystem that triggers the increased temporal resolution of storing the medical parameters and documents in a sensible way, without requiring extensive data communication and storage, wherein the ecosystem can still run fully automatic in the background without any user intervention, except for a user interaction which might be required for the authentication, wherein the high resolution documentation can be provided if required in case of, for instance, legal proceedings.

The medical monitoring system and control method described above with reference to Figs. 1 to 4 allow triggering a detailed data storage process for around a relevant situation which is indicated at least by an authentication of a user. The storage of the detailed medical data, i.e. of the measured medical parameters, by storing them with a higher temporal density than usually required for a medical trend assessment, can be performed by, for instance, a respective patient monitor itself or the detailed medical data can be made available to an external storing unit.

In above described embodiments medical parameters like the heart rate, the breathing rate, the blood pressure, the oxygen saturation, et cetera have been exemplarily mentioned. However, the medical monitoring system can of course also monitor other medical parameters particularly other vital signs. Moreover, although in the above described embodiments the identification unit is adapted to authenticate a user, wherein the medical monitoring system controller is configured to control the medical monitoring system such that the temporal resolution of storing the measured medical parameter in the storing unit depends on the authentication of a user, in another embodiment the identification unit can adapted to carry out a simple identification, for instance, just by asking for entering a user ID without any password request, wherein the medical monitoring system controller can be configured to control the medical monitoring system such that the temporal resolution of storing the measured medical parameter in the storing unit depends on the simple identification of a user.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the processing of the detection signals for measuring the medical parameter, the authentication, the control of the medical monitoring system, et cetera, performed by one or several units or devices can be performed by any other number of units or devices. These procedures, particularly the control of the medical monitoring system in accordance with the control method carried out by the medical monitoring system controller, can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a medical monitoring system controller for controlling a medical monitoring system. The controller is configured to control the medical monitoring system such that the temporal resolution of storing measured medical parameters like blood pressure, heart rate et cetera in a storing unit depends on the identification of a user like a physician or nurse, wherein the identification is preferentially an authentication of the user. In particular, the temporal resolution of storing measured medical parameters can be increased, if a user has been identified. It is therefore not required to store the medical parameter with a relatively high temporal resolution all the time, but this temporal resolution might generally be relatively low and increased, if a user has been identified. This allows for a significant reduction of the required storage capacity.

## Claims

1. A medical monitoring system controller for controlling a medical monitoring system for monitoring a subject, the medical monitoring system (1; 101, 201) comprising a medical parameter measuring unit (4, 6; 104, 106; 204, 206, 211) configured to measure a medical parameter of the subject over time, an identification unit (7) configured to identify a user and a storing unit (8; 208) configured to store the measured medical parameter, wherein the medical monitoring system controller (10; 210) is configured to control the medical monitoring system (1; 101, 201) such that the temporal resolution of storing the measured medical parameter in the storing unit (8; 208) depends on the identification of a user.

2. The medical monitoring system controller as defined by claim 1, wherein the medical monitoring system controller (10; 210) is configured to control the medical monitoring system (1; 101, 201) such that the temporal resolution of storing the measured medical parameter is increased, if a user has been identified.

3. The medical monitoring system controller as defined by any of the preceding claims, wherein the medical monitoring system controller (10; 210) is configured to compare, if a user has been identified, the identified user with a list of users, thereby generating a comparison result, and to control the medical monitoring system (1; 101, 201) such that the temporal resolution of storing the measured medical parameter in the storing unit (8; 208) depends on the comparison result.

4. The medical monitoring system controller as defined by claim 3, wherein the medical monitoring system controller (10; 210) is configured to control the medical monitoring system (1; 101, 201) such that the temporal resolution of storing the measured medical parameter is increased, if the comparison result indicates that the identified user is on the list of users.

5. The medical monitoring system controller as defined by any of the preceding claims, wherein the medical monitoring system controller (10; 210) is configured to determine whether the measured medical parameter fulfils a predefined condition and to control the medical monitoring system (1; 101, 201) such that the temporal resolution of storing the measured medical parameter in the storing unit (8; 208) depends on the fulfilment of the predefined condition.

6. The medical monitoring system controller as defined by any of the preceding claims, wherein the medical parameter measuring unit (4, 6; 104, 106; 204, 206, 211) is configured to measure several medical parameters over time, thereby generating several medical parameter waveforms, wherein the medical monitoring system controller (10; 210) is configured to combine the several medical parameter waveforms for generating a combination result, to determine whether the combination result fulfils a predefined condition, and to control the medical monitoring system (1; 101, 201) such that the temporal resolution of storing the measured medical parameters in the storing unit (8; 208) depends on the fulfilment of the predefined condition.

7. The medical monitoring system as defined by any of the preceding claims, wherein the medical monitoring system controller is configured to control the medical monitoring system such that the temporal resolution of measuring the medical parameter depends on the identification of a user.

8. The medical monitoring system controller as defined by claim 7, wherein the medical monitoring system controller is configured to control the medical monitoring system such that the temporal resolution of measuring the measured medical parameter is increased, if a user has been identified.

9. The medical monitoring system as defined by any of the preceding claims, wherein the medical parameter measuring unit (104, 106) comprises a camera (104) for acquiring images of the subject over time, wherein the medical parameter measuring unit (104, 106) is configured to determine the medical parameter based on the acquired images, wherein the medical monitoring system controller is configured to control the medical monitoring system (101) such that the frequency of acquiring the images depends on the identification of a user.

10. The medical monitoring system controller as defined by any of the preceding claims, wherein the medical monitoring system (201) further comprises a medical report generation unit (212) configured to generate a medical report including the measured medical parameter, wherein the storing unit (208) is configured to store the medical parameter by storing the medical report, wherein the medical monitoring system controller (210) is configured to control the medical monitoring system (201) such that the temporal resolution of the medical parameter included in the medical report depends on the identification of a user.

11. A medical monitoring system for monitoring a subject, the medical monitoring system (1; 101, 201) comprising:
- a medical parameter measuring unit (4, 6; 104, 106; 204, 206, 211) configured to measure a medical parameter of the subject over time,
- an identification unit (7) configured to identify a user,
- a storing unit (8; 208) configured to store the measured medical parameter,
- a medical monitoring system controller (10; 210) configured to control the medical monitoring system as defined by claim 1.

12. The medical monitoring system as defined by claim 11, wherein the medical parameter measuring unit (4, 6; 104, 106; 204, 206, 211) is configured to measure at least one medical parameter of a list consisting of the heart rate, the blood pressure, the oxygen saturation and the breathing rate.

13. A control method for controlling a medical monitoring system for monitoring a subject as defined by claim 11, wherein the medical monitoring system (1; 101, 201) is controlled such that it carries out:
- measuring a medical parameter of the subject over time by a medical parameter measuring unit (4, 6; 104, 106; 204, 206, 211),
- identifying a user by an identification unit (7), and
- storing the measured medical parameter by a storing unit (8; 208) depending on the identification of a user.

14. A computer program for controlling a medical monitoring system for monitoring a subject as defined by claim 11, the computer program comprising program code means for causing the medical monitoring system (1; 101, 201) to carry out the steps of the control method as defined in claim 13, when the computer program is run on the medical monitoring system controller (10; 210) controlling the medical monitoring system (1; 101, 201).
